# EUROPÄISCHE PATENTANMELDUNG

(11) **EP 1 895 011 A1**
(43) Veröffentlichungstag der Anmeldung: **05.03.2008**
(21) Anmeldenummer: 07114910.8
(22) Anmeldetag: 24.08.2007
(51) Int. Cl.: C12P 13/06, C12P 13/08, C07K 14/245

(54) **Verfahren zur Herstellung von L-Aminosäuren unter Verwendung von verbesserten Stämmen der Familie Enterobacteriaceae**

(30) Priorität: 01.09.2006 DE 102006041168
(71) Anmelder: Evonik Degussa GmbH, 40474 Düsseldorf (DE)
(72) Erfinder: Rieping, Mechthild, Dr., 33619 Bielefeld (DE)

(57) **Zusammenfassung**

Die Erfindung betrifft ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man den ytfF-ORF, für das Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

## Beschreibung

Diese Erfindung betrifft ein Verfahren zur Herstellung von L-Aminosäuren, insbesondere L-Threonin, unter Verwendung von rekombinanten Mikroorganismen der Familie Enterobacteriaceae, in denen der offene Leserahmen (ORF) mit der Bezeichnung ytfF abgeschwächt wird, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

### Aufgabe der Erfindung

Die Erfinder haben sich die Aufgabe gestellt, neue Maßnahmen zur verbesserten fermentativen Herstellung von L-Aminosäuren, insbesondere L-Threonin, bereitzustellen.

### Beschreibung der Erfindung

Gegenstand der Erfindung ist ein Verfahren zur fermentativen Herstellung von L-Aminosäuren, insbesondere L-Threonin, unter Verwendung von rekombinanten Mikroorganismen der Familie Enterobacteriaceae, die insbesondere bereits L-Aminosäuren produzieren, und in denen mindestens der offenen Leserahmen ytfF, der für ein putatives Membranprotein eines Aminosäuretransporters kodiert, oder für dessen Genprodukt kodierende Nukleotidsequenzen abgeschwächt, insbesondere ausgeschaltet wird bzw. werden, und die vermehrt L-Aminosäuren, insbesondere L-Threonin, produzieren und in der Zelle oder im Medium anreichern.

Als Ausgangspunkt für den Vergleich dienen jeweils die für den ytfF-ORF nicht rekombinanten Mikroorganismen, die keinen abgeschwächten ytfF-ORF enthalten und an denen die erfindungsgemäße Abschwächung oder Ausschaltung nicht durchgeführt wurde.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt ist L-Threonin.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Gegenstand der Erfindung ist ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man den offenen Leserahmen ytfF oder für dessen Genprodukt kodierende Nukleotidsequenzen oder Allele abschwächt, insbesondere ausschaltet, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem abgeschwächten oder ausgeschalteten offenen Leserahmen (ORF) mit der Bezeichnung ytfF, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, die insbesondere bereits vor Durchführung der Maßnahmen dieser Erfindung L-Aminosäuren über den Eigenbedarf hinaus produzieren, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der den gewünschten ORF, das gewünschte Gen, ein Allel dieses ORFs oder Gens oder Teile davon und/oder einen die Exprimierung des ORFs oder Gens abschwächenden Promotor enthält. Bei diesem Promotor kann es sich um den durch abschwächende Mutation aus der eigenen, upstream des Gens oder ORFs befindlichen regulatorischen Sequenz hervorgegangenen Promotor handeln, oder es wurde ein schwacher Promotor mit dem Gen oder ORF fusioniert.

Als Elternstamm eignen sich Mikroorganismen der Familie Enterobacteriaceae, die insbesondere bereits vor Durchführung der Maßnahmen dieser Erfindung L-Aminosäuren über den Eigenbedarf hinaus produzieren.

Als zum Elternstamm geeignete, insbesondere L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Abschwächung oder Ausschaltung des Gens oder offenen Leserahmens (ORF) ytfF, oder dessen Allelen, vermehrt L-Aminosäuren, insbesondere L-Threonin, produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus dem ebenfalls zur Familie Enterobacteriaceae gehörenden Salmonella typhimurium ist die Nukleotidsequenz für den ytfF-ORF ebenso bekannt (Accession No.: NC_003197 (Region: 4637134-4638099).

Der ytfF-ORF von Escherichia coli K12 wird unter anderem durch folgende Angaben beschrieben:
Das Genprodukt des ytfF-ORFs ist annotiert als putatives Membranprotein eines Aminosäuretransporters der Familie der DM-Transporter (Jack et al., European Journal of Biochemistry 268 (2001) 3620-3639). Es ist beschrieben als ein Mitglied der rhtA-Familie (Livshits et al., Research in Microbiology 154 (2003) 123-135).
Accession No.: U00096 (Region: 4430114-4431088)
Alternativer Genname: b4210

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum ytfF-ORF von Escherichia coli unter der SEQ ID No. 1 und die bekannten Sequenz zum ytfF-ORF von Salmonella typhimurium unter der SEQ ID No. 3 dargestellt. Die von diesen Leserahmen kodierten Proteine sind als SEQ ID No. 2 und SEQ ID No. 4 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des ytfF-ORFs, welche funktionsneutrale Sinnmutationen enthalten, zählen unter anderem solche, die zu höchstens 40 oder zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1 oder SEQ ID No. 3 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1 oder SEQ ID No. 3 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558) .

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin mit Stämmen der Familie Enterobacteriaceae vorteilhaft sein, zusätzlich zur Abschwächung des offenen Leserahmens ytfF, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Rekombinante Mikroorganismen mit einer solchen Verstärkung werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin- und L-Tryptophan-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Eine andere zeitliche Genexpression kann beispielsweise wie bei Walker et al. (Journal of Bacteriology 181: 1269-80 (1999)) beschrieben durch die Verwendung des Wachstumsphasenabhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zweibis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3) : 623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens eines oder mehrere der im folgenden genannten Gene oder für deren Genprodukte kodierende Nukleotidsequenzen oder Allele trägt.

So können beispielsweise gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986); WO 95/11985),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yibD von Escherichia coli (Accession Number AE000439 des National Center for Biotechnology Information (NCBI, Bethesda, MD, USA), DE102004005836.9),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) ytfR von Escherichia coli (Accession Number NC000913 (Region 4449081-4450583) des National Center for Biotechnology Information (NCBI, Bethesda, MD, USA), der auch unter der Bezeichnung ytfS-ORF bekannt ist,
- das Genprodukt des offenen Leserahmens (ORF) ytfT von Escherichia coli (Accession Number NC000913 (Region 4450594-4451619) des National Center for Biotechnology Information (NCBI, Bethesda, MD, USA),
- das Genprodukt des offenen Leserahmens (ORF) yjfF von Escherichia coli (Accession Number NC000913 (Region 4451630-4452601) des National Center for Biotechnology Information (NCBI, Bethesda, MD, USA), und
- das Genprodukt des offenen Leserahmens (ORF) ytfQ von Escherichia coli (Accession Number NC000913 (Region 4447985-4448941) des National Center for Biotechnology Information (NCBI, Bethesda, MD, USA)
verstärkt, insbesondere überexprimiert werden.

Weiterhin kann es für die Produktion von L-Aminosäuren, insbesondere L-Threonin vorteilhaft sein, zusätzlich zur Abschwächung des offenen Leserahmens ytfF, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist und
- das für die Aspartat Ammonium-Lyase kodierende aspA-Gen (WO 03/008603)
abzuschwächen, insbesondere auszuschalten oder die Expression zu verringern.

Weiterhin kann es für die Produktion von L-Aminosäuren, insbesondere L-Threonin vorteilhaft sein, zusätzlich zur Abschwächung des offenen Leserahmens ytfF, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung der L-Aminosäure in der Kulturbrühe. Die Kultur wird solange fortgesetzt, bis sich ein Maximum an L-Aminosäuren bzw. L-Threonin gebildet hat. Dieses Ziel wird normalerweise innerhalb von 10 Stunden bis 160 Stunden erreicht.

Aus der entnommenen Kulturbrühe können die L-Aminosäuren gewonnen, gesammelt oder konzentriert und gegebenenfalls gereinigt werden. Typische Methoden zur Reinigung der L-Aminosäuren sind die Ionenaustauschchromatographie und die Kristallisation. Hierdurch erhält man weitgehend reine L-Aminosäuren.

Es ist ebenfalls möglich aus der entnommenen Kulturbrühe (= Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% oder kleiner 3% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin.

Die vorliegende Erfindung wird nachstehend eingehender anhand der Arbeitsbeispiele erläutert.

Die beschriebenen Minimal- (M9) und Universal-Medien (LB) für E. coli sind von J.H. Miller (A Short course in bacterial genetics (1992), Cold Spring Harbor Laboratory Press) beschrieben. Die Isolation von Plasmid-DNA aus E. coli und sämtliche Techniken in Bezug auf Restriktions-, Ligations-, Klenow- und alkalische Phosphatase-Behandlung werden durchgeführt, wie beschrieben von Sambrook et al. (Molecular cloning. A laboratory manual (1989), Cold Spring Harbor Laboratory Press). Die Transformation von E. coli wird wenn nicht anders beschrieben durchgeführt, wie beschrieben in Chung et al. (Proceedings of the National Academy of Sciences of the United States of America 86: 2172-2175(1989)).

Die Inkubationstemperatur für die Herstellung der Stämme und Transformanten ist wenn nicht anders angegeben 37°C.

### Beispiel 1

Positionsspezifische Mutagenese des ytfF ORF im Stamm E. coli MG442

Der L-Threonin produzierende Stamm E. coli MG 442 ist in der Patentbeschreibung US-A-4,278,765 beschrieben und ist hinterlegt als CMIM B 1628 bei der Russian National Collection for Industrial Microorganisms (VKPM, Moskau, Russland).

### 1.1 Konstruktion der Deletionsmutation des ytfF ORF durch Gen-SOEing

Auf der Basis der Sequenz des für E. coli K12 MG1655 (SEQ ID No. 1, Accession Number U00096 (Region: 4430114-4431088)) bekannten ytfF ORF, werden die folgenden Primer-Oligonukleotide zur Erzeugung der ytfF-Deletionsmutation mit der Polymerasekettenreaktion (PCR) durch das Gen-SOEing-Verfahren (Gene Splicing by Overlap Extension, Horton, Molecular Biotechnology 3: 93-98 (1995)) gewählt:
ytfF_No:
   5' - CAGTACGCGGCCGCTCATGCAGCATGGTCAGGTA - 3`(SEQ ID No. 5) 
ytfF_Ni:

5` - CACGCAATAACCTTCACACTCCAAATTTATAAC ATCGCGCTGTTAGTGATTGG
- 3`

(SEQ ID No. 6)
 
ytfF_Ci:

5` - gttataaatttggagtgtgaaggttattgcgtg CTGGCATCCATTGATCTGTC
- 3`

(SEQ ID No. 7)
 ytfF_Co:
5' - TAGTCGCGGCCGCAGCACTGGAACACGGCGTAT - 3' (SEQ ID No. 8)

Die gezeigten Primer werden von Invitrogen (Karlsruhe, Deutschland) synthetisiert, und die PCR-Reaktion erfolgt durch das Standard-PCR-Verfahren von Innis et al. (PCR Protocols. A Guide to Methods and Applications, 1990, Academic Press). Die für die PCR verwendete chromosomale DNA MG1655 aus E. coli K12 wird gemäß den Herstellerangaben mittels "Qiagen Genomic-tips 100/G" (QIAGEN, Hilden, Germany) isoliert. Die Primer ytfF_No und ytfF_Co enthalten jeweils die Sequenz für eine Spaltstelle der Restriktionsendonuklease NotI, die durch eine Unterstreichung in der oben gezeigten Nukleotidsequenz hervorgehoben ist. Der Primer ytfF_Ni (SEQ ID No. 6) besteht aus zwei Bereichen, von denen einer in der Sequenz des ytfF ORF an die Nukleotide 892 bis 911 (SEQ ID No. 1) bindet, und der andere 5'-terminale Bereich des Primers ytfF_Ni, eine 33 bp Nonsense-Replacement-Sequenz, ist umgekehrt komplementär zum 5'-terminalen Bereich des Primers ytfF_Ci und bindet an die Nukleotide 1 bis 33 des Primers ytfF_Ci (SEQ ID No. 7). Der zweite Bereich des Primers ytfF_Ci bindet in der Sequenz des ytfF ORF an die Nukleotide 1 bis 7 (SEQ ID No. 1) und im benachbarten Stromaufwärtsbereich. Zwischen beiden Bindungsbereichen in der ytfF ORF-Sequenz sind 882 Basenpaare der kodierenden Sequenz des ytfF ORF deletiert.

Mit Hilfe der PCR ermöglichen die Primer ytfF_No und ytfF_Ni die Amplifikation eines DNA-Fragmentes von etwa 0,61 kb Länge, das den 3'-terminalen Bereich des ytfF ORF und den Stromabwärtsbereich enthält, und die Primer ytfF_Ci und ytfF_Co ermöglichen die Amplifikation eines DNA-Fragments von etwa 0,56 kb Länge, das den 5'-terminalen Bereich des ytfF ORF und den Stromaufwärtsbereich enthält. Die Amplifikate werden durch nachfolgende Agarosegelelektrophorese in einem 0,8% Agarosegel untersucht, aus dem Gel isoliert und durch herkömmliche Verfahren gereinigt (High Pure PCR Product Purification Kit, Roche Diagnostics GmbH, Mannheim, Deutschland). Beide Amplifikate werden zusammen als Matrize in einer weiteren PCR-Reaktion mit den Primern ytfF_No und ytfF_Co verwendet. Dadurch erhält man die ytfF Deletionskassette mit etwa 1139 bp Größe, die den ytfF ORF, einschließlich einer 882 bp Deletion und einer 33 bp Nonsense-Replacement-Sequenz, etwa 555 bp des Stomaufwärtsbereichs des ytfF ORF und etwa 544 bp des Stromabwärtsbereichs des ytfF ORF enthält. Die ytfF Deletionskassette hat die in der SEQ ID Nr. 9 gezeigte Sequenz.

Die ytfF-Deletionskassette wird mit der Restriktionsendonuklease NotI gespalten und durch Agarosegelelektrophorese in einem 0,8% Agarosegel identifiziert. Das etwa 1,13 kb lange DNA-Fragment wird aus dem Gel isoliert, durch herkömmliche Verfahren (High Pure PCR Product Purification Kit, Roche Diagnostics GmbH, Mannheim, Deutschland) gereinigt, und zur Ligation mit dem Klonierungsvektor pKO3, beschrieben von Link et al. (Journal of Bacteriology 179: 6228-6237 (1997)), verwendet. Der Vektor pK03 wurde vorher mit der Restriktionsendonuklease NotI gespalten und mit alkalischer Phosphatase (Boehringer, Mannheim) dephosphoryliert, dann mit der ytfF-Deletionskassette gemischt und mit Quick DNA-Ligase (New England BioLabs, Frankfurt, Deutschland) behandelt.

Der Stamm E. coli DH5α (Grant et al.; Proceedings of the National Academy of Sciences USA, 87 (1990) 4645-4649) wird dann mit dem Ligationsansatz (Hanahan, In. DNA cloning. A practical approach. Vol. 1, ILR-Press, Cold Spring Harbor, New York, 1989) transformiert. Die Selektion der plasmidtragenden Zellen erfolgt bei 30°C durch Ausplattieren des Transformationsansatzes auf LB-Agar (Sambrook et al., Molecular Cloning: a laboratory manual. 2nd Ed., Cold Spring Harbor, New York, 1989), der mit 20 mg/l Chloramphenicol angereichert ist.

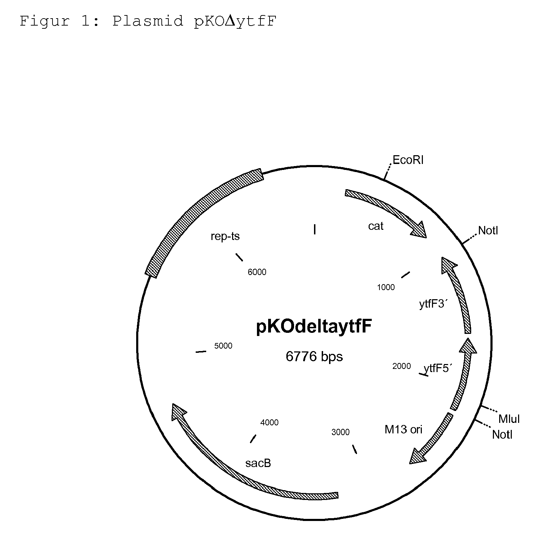

Plasmid-DNA wird aus einer Transformante mittels QIAprep Spin Miniprep Kit von Qiagen isoliert und durch Restriktionsspaltung mit den Enzymen EcoRI und MluI und anschließende Agarosegelelektrophorese überprüft. Das Plasmid wird als pKOΔytfF bezeichnet (Figur 1).

### 1.2 Stellenspezifische Mutagenese des ytfF ORF im Stamm E. coli MG442

Für den Austausch des chromosomalen ytfF ORF durch das plasmidkodierte Deletionskonstrukt, wird MG442 mit dem Plasmid pKOΔytfF transformiert. Der Genaustausch erfolgt durch das Selektionsverfahren, beschrieben von Link et al. (Journal of Bacteriology 179: 6228-6237 (1997)), und wird durch Standard-PCR-Verfahren (Innis et al. (1990) PCR Protocols. A guide to methods and applications, Academic Press) mit folgenden Oligonukleotid-Primern überprüft:
ytfF No:
   5' - CAGTACGCGGCCGCTCATGCAGCATGGTCAGGTA - 3' (SEQ ID No. 5)
ytfF Co:
   5' - TAGTCGCGGCCGCAGCACTGGAACACGGCGTAT - 3' (SEQ ID No. 8)

Nach dem Austausch ist die Form des in SEQ ID No. 9 gezeigten ΔytfF-Allels von Position 524 bis 647 in MG442 zugegen. Der erhaltene Stamm hat die Bezeichnung MG442ΔytfF.

### Beispiel 2

### Herstellung von L-Threonin mit dem Stamm MG442ΔytfF

MG442ΔytfF wird auf Minimalmedium mit der folgenden Zusammensetzung vermehrt: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar. Die Bildung von L-Threonin wird in 10 ml Batch-Kulturen, enthalten in 100 ml Erlenmeyerkolben, überprüft. Zu diesem Zweck werden 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose beeimpft und der Ansatz wird für 16 Std. bei 37°C und 180 U/min auf einem ESR-Inkubator von der Kühner AG (Birsfelden, Schweiz) inkubiert. 250 µl dieser Vorkultur werden in 10 ml Produktionsmedium überimpft (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose), und der Ansatz wird für 48 Std. bei 37°C inkubiert. Nach der Inkubation wird die optische Dichte (OD) der Kultursuspension mit einem LP2W Photometer von Dr. Lange (Düsseldorf, Deutschland) bei einer Messwellenlänge von 660 nm bestimmt.

Die Konzentration des entstandenen L-Threonin wird dann in dem steril-filtrierten Kulturüberstand mit einem Aminosäureanalysator von Eppendorf-BioTronik (Hamburg, Deutschland) durch Ionenaustauschchromatographie und Nachsäulenreaktion unter Ninhydrinnachweis bestimmt.

Das Ergebnis des Experiments ist in tabelle 1 gezeigt.

**Tabelle 1**

| Stamm | OD (660 nm) | L-Threonin g/l |
|---|---|---|
| MG442 | 5,9 | 1,35 |
| MG442ΔytfF | 5,7 | 1,80 |

### Beispiel 3

Positionsspezifische Mutagenese des thrB-Gens im Stamm E. coli MG442

Der L-homoserinproduzierende Stamm E. coli MG442ΔthrB ist vom Stamm MG442 hergeleitet, der in der Patentbeschreibung US-A-4,278,765 beschrieben ist und als CMIM B-1628 bei der Russian National Collection for Industrial Microorganisms (VKPM, Moskau, Russland) hinterlegt ist.

### 3.1 Konstruktion der Deletionsmutation des thrB-Gens durch Gen-SOEing

Auf der Basis der Sequenz des thrB-Gens, das für E. coli K12 MG1655 (SEQ ID No. 10, Accession Number U00096) bekannt ist, werden die folgenden Primer-Oligonukleotide für die Erzeugung der thrB-Deletionsmutation mittels Polymerase kettenreaktion (PCR) durch das Gen-SOEing-Verfahren (Gene Splicing by Overlap Extension, Horton, Molecular Biotechnology 3: 93-98 (1995)) gewählt:
thrB_No: 5' - CAGTACGCGGCCGCTGCAGGTGATGAATTGATGAAG - 3' (SEQ ID No. 12)
thrB_Ni: 5' - CACGCAATAACCTTCACACTCCAAATTTATAACTAACCATGTCAGACTCCTAACT - 3' (SEQ ID No. 13)
thrB_Ci: 5' - GTTATAAATTTGGAGTGTGAAGGTTATTGCGTGCGACCTTGTTCGCTCTGTGT - 3' (SEQ ID No. 14)
thrB_Co: 5' - TAGTCGCGGCCGCGCCTCGTGGATAGAGGATAACC - 3' (SEQ ID No. 15)

Die gezeigten Primer werden von Invitrogen (Karlsruhe, Deutschland) synthetisiert, und die PCR-Reaktion erfolgt durch das Standard-PCR-Verfahren von Innis et al. (PCR Protocols. A Guide to Methods and Applications, 1990, Academic Press). Die für die PCR verwendete chromosomale DNA MG1655 aus E. coli K12 wird gemäß den Herstellerangaben mittels "Qiagen Genomic-tips 100/G" (QIAGEN, Hilden, Deutschland) isoliert. Die Primer thrB_No und thrB_Co enthalten jeweils die Sequenz für eine Spaltstelle der Restriktionsendonuklease NotI, die durch eine Unterstreichung in der oben gezeigten Nukleotidsequenz hervorgehoben ist. Der Primer thrB_Ni (SEQ ID No. 13) besteht aus zwei Bereichen, von denen einer in der Sequenz des thrB-Gens an die Nukleotide 579 bis 600 (SEQ ID No. 10) bindet, und der andere 5'-terminale Bereich des Primers thrB_Ni, eine 33 bp Nonsense-Replacement-Sequenz, ist umgekehrt komplementär zum 5'-terminalen Bereich des Primers thrB_Ci und bindet an die Nukleotide 1 bis 33 des Primers thrB_Ci (SEQ ID No. 14). Der zweite Bereich des Primers thrB_Ci bindet in der Sequenz des thrB-Gens an die Nukleotide 1384 bis 1403 (SEQ ID No. 10). Zwischen beiden Bindungsbereichen in der thrB-Gen-Sequenz sind 783 Basenpaare der kodierenden Sequenz des thrB-Gens deletiert.

Mit Hilfe der PCR ermöglichen die Primer thrb_No und thrB_Ni die Amplifikation eines DNA-Fragmentes von etwa 0,65 kb Länge, das den 5'-terminalen Bereich des thrB-Gens und den Stromaufwärtsbereich enthält, und die Primer thrB_Ci und thrB_Co ermöglichen die Amplifikation eines DNA-Fragments von etwa 0,67 kb Länge, das den 3'-terminalen Bereich des thrB-Gens und den Stromabwärtsbereich enthält. Die Amplifikate werden durch nachfolgende Agarosegelelektrophorese in einem 0,8% Agarosegel untersucht, aus dem Gel isoliert und durch herkömmliche Verfahren gereinigt (High Pure PCR Product Purification Kit, Roche Diagnostics GmbH, Mannheim, Deutschland). Beide Amplifikate werden zusammen als Matrize in einer weiteren PCR-Reaktion mit den Primern thrB_No und thrB_Co verwendet. Dadurch erhält man die thrB-Deletionskassette mit etwa 1282 bp Größe, die das thrB-Gen, einschließlich einer 783 bp Deletion und einer 33 bp Nonsense-Replacement-Sequenz, etwa 600 bp des Stomaufwärtsbereichs des thrB-Gens und etwa 480 bp des Stromabwärtsbereichs des thrB-Gens enthält.

Die thrB-Deletionskassette wird mit der Restriktionsendonuklease NotI gespalten und durch Agarosegelelektrophorese in einem 0,8% Agarosegel identifiziert. Das etwa 1,26 kb lange DNA-Fragment wird aus dem Gel isoliert, durch herkömmliche Verfahren (High Pure PCR Product Purification Kit, Roche Diagnostics GmbH, Mannheim, Deutschland) gereinigt, und zur Ligation mit dem Klonierungsvektor pK03, beschrieben von Link et al. (Journal of Bacteriology 179: 6228-6237 (1997)) verwendet.

Der Vektor pKO3 wurde vorher mit der Restriktionsendonuklease NotI gespalten und mit alkalischer Phosphatase (Boehringer, Mannheim) dephosphoryliert, dann mit der thrB-Deletionskassette gemischt und mit Quick DNA-Ligase (New England BioLabs, Frankfurt, Deutschland) behandelt.

Der Stamm E. coli DH5α (Grant et al.; Proceedings of the National Academy of Sciences USA, 87 (1990) 4645-4649) wird dann mit dem Ligationsansatz (Hanahan, In. DNA cloning. A practical approach. Vol. 1, ILR-Press, Cold Spring Harbor, New York, 1989) transformiert. Die Selektion der plasmidtragenden Zellen erfolgt bei 30°C durch Ausplattieren des Transformationsansatzes auf LB-Agar (Sambrook et al., Molecular Cloning: a laboratory manual. 2nd Ed., Cold Spring Harbor, New York, 1989), der mit 20 mg/l Chloramphenicol angereichert ist.

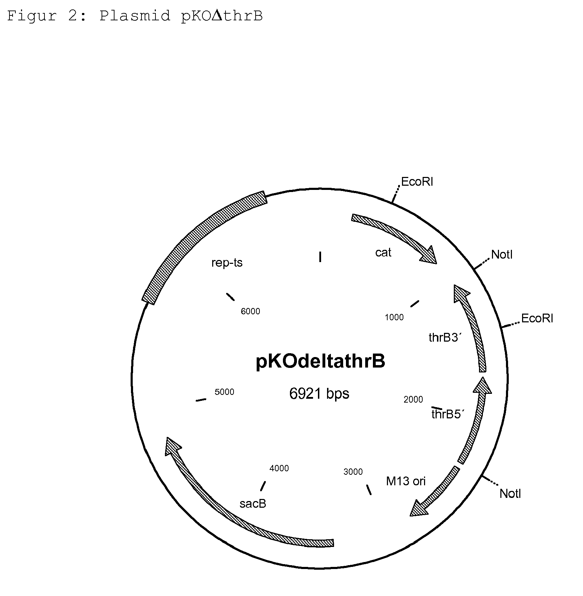

Plasmid-DNA wird aus einer Transformante mittels QIAprep Spin Miniprep Kit von Qiagen isoliert und durch Restriktionsspaltung mit dem Enzym EcoRI und anschließende Agarosegelelektrophorese überprüft. Das Plasmid wird als pkoΔthrB bezeichnet (Figur 2).

### 3.2 Stellenspezifische Mutagenese des thrB-Gens im Stamm E. coli MG442

Der L-Threonin-produzierende Stamm E. coli MG442 ist in der Patentbeschreibung US-A-4,278,765 beschrieben und ist als CMIM B 1628 bei der Russian National Collection for Industrial Microorganisms (VKPM, Moskau, Russland) hinterlegt.

Für den Austausch des chromosomalen thrB-Gens durch das plasmidkodierte Deletionskonstrukt, wird MG442 mit dem Plasmid pKOΔthrB transformiert. Der Genaustausch erfolgt durch das Selektionsverfahren, beschrieben von Link et al. (Journal of Bacteriology 179: 6228-6237 (1997)) und wird durch Standard-PCR-Verfahren (Innis et al. (1990) PCR Protocols. A guide to methods and applications, Academic Press) mit folgenden Oligonukleotid-Primern überprüft:
thrB_No: 5` - CAGTACGCGGCCGCTGCAGGTGATGAATTGATGAAG - 3` (SEQ ID No. 12)
thrB_Co: 5' - TAGTCGCGGCCGCGCCTCGTGGATAGAGGATAACC - 3' (SEQ ID No. 15)

Nach dem Austausch ist die Form des in SEQ ID No. 16 gezeigten ΔthrB-Allels in MG442 zugegen. Der erhaltene Stamm hat die Bezeichnung MG442ΔthrB.

### Beispiel 4

In-vivo-Mutagenese des Stamms MG442ΔthrB

Ausgehend von MG442ΔthrB, werden nach Inkubation bei 37°C in L-Homoserin (Sigma, Taufkirchen, Deutschland) enthaltendem Minimalagar, zu dem 2 g/l Glucose und 2 g/l Threonin und Isoleucin gegeben wurden, spontane Mutanten isoliert, die auf 2 g/l L-Homoserin wachsen können. Eine selektierte Mutante hat die Bezeichnung MG442ΔthrBHom2.

### Beispiel 5

### Stellenspezifische Mutagenese des ytfF ORF im Stamm E. coli MG442ΔthrBHom2

Für den Austausch des chromosomalen ytfF ORF gegen das plasmidkodierte Deletionskonstrukt, wird MG442ΔthrBHom2 mit dem Plasmid pKOΔytfF transformiert (siehe Beispiel 1). Der Genaustausch erfolgt durch das Selektionsverfahren, beschrieben von Link et al. (Journal of Bacteriology 179: 6228-6237 (1997)) und wird durch Standard-PCR-Verfahren (Innis et al. (1990) PCR Protocols. A guide to methods and applications, Academic Press) mit den folgenden Oligonukleotid-Primern überprüft:
ytfF No:
   CAGTACGCGGCCGCTCATGCAGCATGGTCAGGTA (SEQ ID No. 5)
ytfF Co:
   TAGTCGCGGCCGCAGCACTGGAACACGGCGTAT (SEQ ID No. 8)

Nach dem Austausch ist die Form des in SEQ ID No. 9 gezeigten ΔytfF-Allels von Position 524 to 647 in MG442ΔthrBHom2 zugegen. Der erhaltene Stamm hat die Bezeichnung MG442ΔthrBHom2ΔytfF.

### Beispiel 6

Konstruktion des Expressionsplasmids pTrc99AthrA

Das thrA-Gen aus E. coli K12 wird mit der Polymerasekettenreaktion (PCR) und synthetischen Oligonukleotiden amplifiziert. Ausgehend von der Nukleotidsequenz des thrA-Gens in E. coli K12 MG1655 (Accession Number U00096, Blattner et al. (Science 277: 1453-1462 (1997)), werden folgende PCR-Primer synthetisiert (MWG Biotech, Ebersberg, Deutschland):
thrA1: 5` - AAGGTAACGAGGTAACAACC - 3` (SEQ ID No. 17)
thrA2: 5` - TCAAACCCGACGCTCATATT - 3' (SEQ ID No. 18)

Chromosomale E. coli K12 MG1655 DNA, die für die PCR eingesetzt wird, wird gemäß den Herstellerangaben mit "Qiagen Genomic-tips 100/G" (QIAGEN, Hilden, Deutschland) isoliert. Ein DNA-Fragment mit etwa 2537 bp Größe kann mit den spezifischen Primern unter Standard-PCR-Bedingungen (Innis et al. (1990) PCR Protocols. A Guide to Methods and Applications, Academic Press) mit Pfu-DNA-Polymerase (Promega Corporation, Madison, USA) amplifiziert werden.

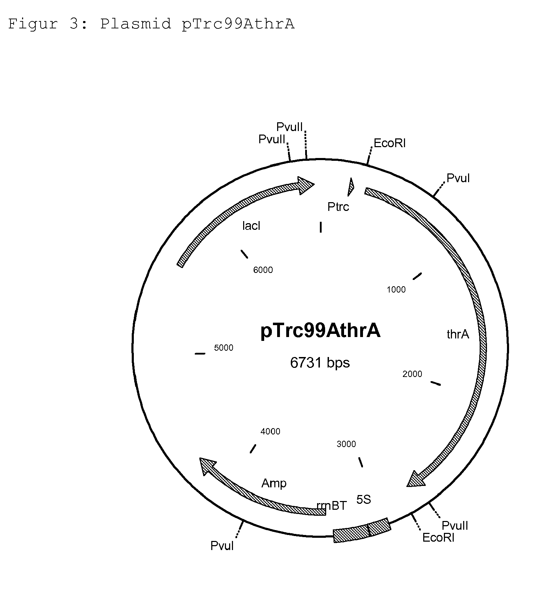

Das PCR-Produkt wird mit dem Vektor pCR-Blunt II-TOPO (Zero Blunt TOPO PCR Cloning Kit, Invitrogen, Groningen, Die Niederlande) gemäß den Herstellerangaben ligiert und in den Stamm E. coli TOP10 transformiert. Die Selektion auf plasmidtragende Zellen erfolgt auf LB-Agar, zu dem 50 µg/ml Kanamycin zugegeben wurde. Nach Isolation der Plasmid-DNA wird der Vektor pCR-Blunt II-TOPO-thrA mit dem Restriktionsenzym EcoRI gespalten und nach der Trennung in einem 0,8% Agarosegel mittels QIAquick Gel Extraction Kit (QIAGEN, Hilden, Deutschland), wird das thrA-Fragment isoliert. Der Vektor pTrc99A (Pharmacia Biotech, Uppsala, Schweden) wird mit dem Enzym EcoRI gespalten und mit dem isolierten thrA-Fragment ligiert. Der Stamm E. coli XL1-Blue MRF' (Stratagene, La Jolla, USA) wird mit dem Ligationsansatz transformiert und plasmidtragende Zellen werden auf LB-Agar selektiert, zu dem 50 µg/ml Ampicillin gegeben wurde. Eine erfolgreiche Klonierung kann nach Plasmid-DNA-Isolation durch Kontrollspaltung mit den Enzymen PvuI and PvuII nachgewiesen werden. Das Plasmid hat die Bezeichnung pTrc99AthrA (Figur 3).

### Beispiel 7

### Herstellung von L-Homoserin mit dem Stamm MG442ΔthrBHom2ΔytfF/pTrc99AthrA

Die Stämme MG442ΔthrBHom2ΔytfF und MG442ΔthrBHom2 werden mit dem Expressionsplasmid pTrc99AthrA, beschrieben in Beispiel 6, transformiert, und plasmidtragende Zellen werden auf LB-Agar mit 50 µg/ml Ampicillin selektiert. Die Stämme MG442ΔthrBHom2ΔytfF/pTrc99AthrA und MG442ΔthrBHom2/pTrc99AthrA werden auf diese Weise gebildet. Ausgewählte Einzelkolonien werden dann weiter auf Minimalmedium mit der folgenden Zusammensetzung vermehrt: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin, 10 mg/l L-Threonin, 10 mg/l L-Isoleucin.

Die Bildung von L-Homoserin durch die Stämme MG442ΔthrBHom2ΔytfF/pTrc99AthrA und MG442ΔthrBHom2/pTrc99AthrA wird in 10 ml Batch-Kulturen, die sich in 100 ml Erlenmeyerkolben befinden, überprüft. Zu diesem Zweck werden 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose und 50 mg/l Ampicillin beimpft, und der Ansatz wird für 16 Std. bei 37°C und 180 U/min in einem ESR-Inkubator von der Kühner AG (Birsfelden, Schweiz) inkubiert. 250 µl dieser Vorkultur werden in 10 ml Produktionsmedium (25 g/l (NH₄)₂SO₄, 2 g/l KH₂PO₄ ,1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin, 10 mg/l L-Threonin, 10 mg/l L-Isoleucin und 5 mg/l Thiamin) überimpft, und der Ansatz wird 72 Std. bei 37°C inkubiert. Nach der Inkubation wird die optische Dichte (OD) der Kultursuspension mit einem LP2W-Photometer von Dr. Lange (Berlin, Deutschland) bei einer Messwellenlänge von 660 nm bestimmt.

Die Konzentration des entstandenen L-Homoserins wird dann in dem sterilfiltrierten Kulturüberstand mit einem Aminosäureanalysator von Eppendorf-BioTronik (Hamburg, Deutschland) durch Ionenaustauschchromatographie und Nachsäulenreaktion mit Ninhydrinnachweis bestimmt.

Das Ergebnis des Experimentes ist in der Tabelle 2 gezeigt.

**Tabelle 2**

| Stamm | OD (660 nm) | L-Homoserin g/l |
|---|---|---|
| MG442ΔthrBHom2/ pTrc99AthrA | 4,0 | 1,50 |
| MG442ΔthrBHom2Δyt fF/ pTrc99AthrA | 4,1 | 1,65 |

Kurze Beschreibung der Figur:
- Figur 1: pKOΔytfF (= pKOdeltaytfF)
- Figur 2: pKOΔthrB (= pKOdeltathrB)
- Figur 3: pTrc99AthrA

Die Längendaten verstehen sich als Näherungsdaten. Die verwendeten Abkürzungen und Bezeichnungen haben die folgende Bedeutung:
- Amp: Ampicillinresistenzgen
- cat: Chloramphenicolresistenzgen
- lacI: Gen für das Repressorprotein des trc-Promotors
- Ptrc: trc-Promotorregion, IPTG-induzierbar
- thrA: Kodierende Region des thrA-Gens
- 5S: 5S rRNA_Region
- rrnBT: rRNA-Terminatorregion
- M13 ori: Replikationsursprung
- sacB: sacB-Gen
- thrB5': Teil der 5'-Region des thrB-Gens und der stromaufwärts befindlichen Region
- thrB3': Teil der 3'-Region des thrB-Gens und der stromabwärts befindlichen Region
- ytfF5' : Teil der 5'-Region des ytfF ORF und der stromaufwärts befindlichen Region
- YtfF3': Teil der 3'-Region des ytfF ORF und der stromabwärts befindlichen Region

Die Abkürzungen für die Restriktionsenzyme haben die folgende Bedeutung
- EcoRI: Restriktionsendonuklease aus Escherichia coli RY13
- PvuI: Restriktionsendonuklease aus Proteus vulgaris
- MluI:Restriktionsendonuklease aus Micrococcus luteus
- NotI:Restriktionsendonuklease aus Nocardia otitidiscaviarum

## Patentansprüche

1. Verfahren zur Herstellung von L-Aminosäuren, **dadurch gekennzeichnet, dass** man folgende Schritte durchführt:
a) Fermentation der die gewünschte L-Aminosäure produzierenden Mikroorganismen der Familie Enterobacteriaceae, in denen man den ytfF-ORF, der für ein putatives Membranprotein eines Aminosäuretransporters kodiert, oder die für das Genprodukt kodierende Nukleotidsequenz abschwächt, insbesondere ausschaltet, und
b) Anreicherung der gewünschten L-Aminosäure im Medium oder in den Zellen der Mikroorganismen.

2. Verfahren gemäß Anspruch 1, **dadurch gekennzeichnet, dass** man die gewünschte L-Aminosäure isoliert, wobei gegebenenfalls Bestandteile der Fermentationsbrühe und/oder die Biomasse in ihrer Gesamtheit oder Anteilen (> 0 bis 100 %) davon im Produkt verbleiben.

3. Verfahren gemäß den Ansprüchen 1 oder 2, **dadurch gekennzeichnet, dass** man zur Herstellung von L-Aminosäuren, insbesondere L-Threonin, oder diese Verbindungen enthaltenden Futtermitteladditiven durch Fermentation Mikroorganismen der Familie Enterobacteriaceae, **dadurch gekennzeichnet, dass** man in diesen den ytfF-ORF oder für dessen Genprodukt kodierende Nukleotidsequenzen abschwächt, insbesondere ausschaltet, einsetzt und das gewünschte Produkt isoliert.

4. Verfahren gemäß den Ansprüchen 1, 2, oder 3, **dadurch gekennzeichnet, dass** man Mikroorganismen einsetzt, in denen die Aktivität oder Konzentration des ytfF Genprodukts auf 0 bis 75% der Aktivität oder Konzentration des Wildtyp-Proteins oder im Elternstamm senkt.

5. Verfahren gemäß den Ansprüchen 1 bis 4, **dadurch gekennzeichnet, dass** man Mikroorganismen einsetzt, in denen man zusätzlich weitere Gene des Biosyntheseweges der gewünschten L-Aminosäure verstärkt.

6. Verfahren gemäß den Ansprüchen 1 bis 5, **dadurch gekennzeichnet, dass** man Mikroorganismen einsetzt, in denen die Stoffwechselwege zumindest teilweise ausgeschaltet sind, die die Bildung der gewünschten L-Aminosäure verringern.

7. Verfahren gemäß den Ansprüchen 1, 2, oder 3, **dadurch gekennzeichnet, dass** man die Expression des Polynukleotids, das für das Produkt des ytfF-ORFs kodiert, abschwächt, insbesondere ausschaltet.

8. Verfahren gemäß den Ansprüchen 1, 2 oder 3, **dadurch gekennzeichnet, dass** man die regulatorischen und/oder katalytischen Eigenschaften des Polypeptids (Enzymproteins) verringert, für das das Polynukleotid des ytfF-ORFs kodiert.

9. Verfahren gemäß den Ansprüchen 1 bis 8, **dadurch gekennzeichnet, dass** man zur Herstellung der gewünschten L-Aminosäuren Mikroorganismen der Familie Enterobacteriaceae fermentiert, in denen man zusätzlich gleichzeitig eines oder mehrere der Gene, ausgewählt aus der Gruppe:
9.1 mindestens ein Gen des für die Aspartatkinase, die Homoserin-Dehydrogenase, die Homoserinkinase und die Threoninsynthase kodierenden thrABC-Operons,
9.2 das für die Pyruvat-Carboxylase kodierende pyc-Gen von Corynebacterium glutamicum,
9.3 das für die Phosphoenolpyruvat-Synthase kodierende pps-Gen,
9.4 das für die Phosphoenolpyruvat-Carboxylase kodierende ppc-Gen,
9.5 die für die Untereinheiten der Pryridin-Transhydrogenase kodierenden Gene pntA und pntB,
9.6 das für das Threoninresistenz vermittelnde Protein kodierende Gen rhtC,
9.7 das für das Threonin-Export-Carrier-Protein kodierende thrE-Gen aus Corynebacterium glutamicum,
9.8 das für die Glutamat-Dehydrogenase kodierende gdhA-Gen,
9.9 das für die Phosphohistidin-Protein-Hexose-Phosphotransferase kodierende ptsH-Gen,
9.10 das für das Enzym I des Phosphotransferase-Systems kodierende ptsI-Gen,
9.11 das für die Glucose-spezifische IIA Komponente kodierende crr-Gen,
9.12 das für die Glucose-spezifische IIBC Komponente kodierende ptsG-Gen,
9.13 das für die Cystein-Synthase A kodierende cysK-Gen,
9.14 das für den Regulator des cys-Regulons kodierende cysB-Gen,
9.15 das für das Flavoprotein der NADPH-Sulfit-Reduktase kodierende cysJ-Gen,
9.16 das für das Hämoprotein der NADPH-Sulfit-Reduktase kodierende cysI-Gen,
9.17 das für die Adenylylsulfat-Reduktase kodierende cysH-Gen,
9.18 das für die Decarboxylase Untereinheit der 2-Ketoglutarat Dehydrogenase kodierende sucA-Gen,
9.19 das für die Dihydrolipoyltranssuccinase E2 Untereinheit der 2-Ketoglutarat Dehydrogenase kodierende sucB-Gen,
9.20 das für die β-Untereinheit der Succinyl-CoA Synthetase kodierende sucC-Gen,
9.21 das für die α-Untereinheit der Succinyl-CoA Synthetase kodierende sucD-Gen,
9.22 das Genprodukt des offenen Leserahmens (ORF) yibD von Escherichia coli,
9.23 das Genprodukt des offenen Leserahmens (ORF) yjcG von Escherichia coli,
9.24 das Genprodukt des offenen Leserahmens (ORF) ytfR von Escherichia coli,
9.25 das Genprodukt des offenen Leserahmens (ORF) ytfT von Escherichia coli,
9.26 das Genprodukt des offenen Leserahmens (ORF) yjfF von Escherichia coli, und
9.27 das Genprodukt des offenen Leserahmens (ORF) ytfQ von Escherichia coli,
verstärkt, insbesondere überexprimiert.

10. Verfahren gemäss den Ansprüchen 1 bis 9, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
10.1 das für die Threonin-Dehydrogenase kodierende tdh-Gen,
10.2 das für die Malat-Dehydrogenase kodierende mdh-Gen,
10.3 das Genprodukt des offenen Leserahmens (ORF) yjfA von Escherichia coli,
10.4 das Genprodukt des offenen Leserahmens (ORF) ytfP von Escherichia coli,
10.5 das für die Phosphoenolpyruvat-Carboxykinase kodierende pckA-Gen,
10.6 das für die Pyruvat-Oxidase kodierende poxB-Gen,
10.7 das für den DgsA-Regulator des Phosphotransferase-Systems kodierende dgsA-Gen,
10.8 das für den Fructose-Repressor kodierende fruR-Gen,
10.9 das für den Sigma³⁸-Faktor kodierende rpoS-Gen, und
10.10 das für die Aspartat Ammonium-Lyase kodierende aspA-Gen
abschwächt, insbesondere ausschaltet oder die Expression verringert.

11. Verfahren gemäss den Ansprüchen 1 bis 10, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Arginin, L-Homoserin, L-Tryptophan und L-Threonin herstellt.

12. Verfahren gemäss den Ansprüchen 1 bis 10, **dadurch gekennzeichnet, dass** man L-Aminosäuren, ausgewählt aus der Gruppe L-Isoleucin, L-Valin, L-Methionin, L-Homoserin, L-Lysin, L-Tryptophan und L-Threonin herstellt.

13. Rekombinante L-Aminosäure produzierende Mikroorganismen, in denen der ytfF-ORF, der für ein putatives Membranprotein eines Aminosäuretransporters kodiert, oder die für das Genprodukt kodierende Nukleotidsequenz abgeschwächt, insbesondere ausgeschaltet ist.

14. Mikroorganismus gemäss Anspruch 13, **dadurch gekennzeichnet, dass** sie durch Transformation, Transduktion, Konjugation oder eine Kombination dieser Methoden hergestellt werden, mit einem Vektor, der den ytfF-ORF, ein Allel dieses ORFs oder Teile davon und/oder einen abgeschwächten Promotor enthält.

15. Mikroorganismen gemäss den Ansprüchen 13 und 14, **dadurch gekennzeichnet, dass** man zur Erzielung der Abschwächung
a) die Promotor- und Regulationsregion oder die Ribosomenbindungsstelle stromaufwärts des ytfF-ORFs mutiert, oder
b) Expresssionskassetten oder Promotoren stromaufwärts des ytfF-ORFs einbaut.

16. Mikroorganismen gemäss den Ansprüchen 13 bis 15, **dadurch gekennzeichnet, dass** die Expression des ytfF-ORFs unter der Kontrolle eines die Expression des ORFs abschwächenden Promotors steht.

17. Mikroorganismen gemäss den Ansprüchen 13 bis 16, **dadurch gekennzeichnet, dass** durch die Abschwächung des ytfF-ORFs die Konzentration oder Aktivität des ytfF-Genproduktes (Proteins) auf mindestens 0% - 75% verringert sind, bezogen auf die Aktivität oder Konzentration des Genproduktes im für den ytfF-ORF nicht rekombinanten Mikroorganismus oder Elternstamm.

18. Mikroorganismen gemäss den Ansprüchen 13 bis 17, **dadurch gekennzeichnet, dass** die Mikroorganismen aus den Gattungen Escherichia, Erwinia, Providencia und Serratia ausgewählt sind.

19. Mikroorganismen gemäss den Ansprüchen 13 bis 18, **dadurch gekennzeichnet, dass** sie L-Threonin oder L-Homoserin produzieren.


SEQUENZPROTOKOLL

<110>  Degussa GmbH

<120>  Verfahren zur Herstellung von L-Aminosäuren unter Verwendung
       von verbesserten Stämmen der Familie Enterobacteriaceae

<130>  xxx

<160>  18

<170>  PatentIn Version 3.3

<210>  1
<211>  975
<212>  DNA
<213>  Escherichia coli

<220>
<221>  CDS
<222>  (1)..(975)
<223>  codierende Region von ytfF

<400>  1
atg cca gta atg att agc ggc gtg ctg tac gcc ctg tta gca ggg ttg       48
Met Pro Val Met Ile Ser Gly Val Leu Tyr Ala Leu Leu Ala Gly Leu
1               5                   10                  15

atg tgg ggg ctt att ttt gtc ggg ccg ttg atc gtg ccg gaa tac ccg       96
Met Trp Gly Leu Ile Phe Val Gly Pro Leu Ile Val Pro Glu Tyr Pro
            20                  25                  30

gcg atg ttg cag tcg atg ggg cgt tat ctg gcg tta ggg tta att gcg      144
Ala Met Leu Gln Ser Met Gly Arg Tyr Leu Ala Leu Gly Leu Ile Ala
        35                  40                  45

ctg ccc att gcc tgg ctg gga cgc gtg cgt ctg cgt cag ttg gcg cgt      192
Leu Pro Ile Ala Trp Leu Gly Arg Val Arg Leu Arg Gln Leu Ala Arg
    50                  55                  60

cgc gac tgg ctt acc gcc ttg atg ctc act atg atg ggc aac ctc atc      240
Arg Asp Trp Leu Thr Ala Leu Met Leu Thr Met Met Gly Asn Leu Ile
65                  70                  75                  80

tat tac ttc tgc ctt gcc agt gcc att caa cgt act ggc gcg cct gtt      288
Tyr Tyr Phe Cys Leu Ala Ser Ala Ile Gln Arg Thr Gly Ala Pro Val
                85                  90                  95

tcc acg atg att atc ggc acc ctg ccg gtg gtc att cct gtc ttt gcc      336
Ser Thr Met Ile Ile Gly Thr Leu Pro Val Val Ile Pro Val Phe Ala
            100                 105                 110

aat ctg ctt tat agc cag cgc gac ggc aaa ctc gcg tgg gga aaa ctc      384
Asn Leu Leu Tyr Ser Gln Arg Asp Gly Lys Leu Ala Trp Gly Lys Leu
        115                 120                 125

gcc cca gca ctg att tgt att ggc atc ggc ctg gcg tgt gtg aat att      432
Ala Pro Ala Leu Ile Cys Ile Gly Ile Gly Leu Ala Cys Val Asn Ile
    130                 135                 140

gct gag tta aac cac gga ctc ccc gat ttt gac tgg gca cgt tat acc      480
Ala Glu Leu Asn His Gly Leu Pro Asp Phe Asp Trp Ala Arg Tyr Thr
145                 150                 155                 160

tct ggc atc gtg ctg gcg tta gtt tcc gtg gtc tgc tgg gcg tgg tat      528
Ser Gly Ile Val Leu Ala Leu Val Ser Val Val Cys Trp Ala Trp Tyr
                165                 170                 175

gcc ctg cgc aac gcc cgc tgg ctg cgg gaa aat ccc gac aaa cat ccg      576
Ala Leu Arg Asn Ala Arg Trp Leu Arg Glu Asn Pro Asp Lys His Pro
            180                 185                 190

atg atg tgg gca aca gcg cag gcg ctg gtc aca ctg ccg gtt tca ctt      624
Met Met Trp Ala Thr Ala Gln Ala Leu Val Thr Leu Pro Val Ser Leu
        195                 200                 205

atc ggc tat ctc gtc gct tgt tac tgg ctg aat acg caa acg ccg gac      672
Ile Gly Tyr Leu Val Ala Cys Tyr Trp Leu Asn Thr Gln Thr Pro Asp
    210                 215                 220

ttc tcc cta ccc ttt ggc ccc cgt ccg ctg gtg ttt att agt ctg atg      720
Phe Ser Leu Pro Phe Gly Pro Arg Pro Leu Val Phe Ile Ser Leu Met
225                 230                 235                 240

gtt gcg ata gcc gtg ctt tgc tca tgg gtt ggc gca ctc tgc tgg aac      768
Val Ala Ile Ala Val Leu Cys Ser Trp Val Gly Ala Leu Cys Trp Asn
                245                 250                 255

gtc gcc agc cag cta tta ccg aca gtg att ctc ggg ccg ctg att gtt      816
Val Ala Ser Gln Leu Leu Pro Thr Val Ile Leu Gly Pro Leu Ile Val
            260                 265                 270

ttc gaa acg ctg gca ggt ttg ctg tac acc ttt tta ctc cgc cag caa      864
Phe Glu Thr Leu Ala Gly Leu Leu Tyr Thr Phe Leu Leu Arg Gln Gln
        275                 280                 285

atg ccg ccg cta atg acg ctg agc ggt atc gcg ctg tta gtg att ggc      912
Met Pro Pro Leu Met Thr Leu Ser Gly Ile Ala Leu Leu Val Ile Gly
    290                 295                 300

gtg gtc atc gcg gtc aga gca aaa cca gaa aaa cct tta act gaa tct      960
Val Val Ile Ala Val Arg Ala Lys Pro Glu Lys Pro Leu Thr Glu Ser
305                 310                 315                 320

gtc tca gaa agt tga                                                  975
Val Ser Glu Ser

<210>  2
<211>  324
<212>  PRT
<213>  Escherichia coli

<400>  2

Met Pro Val Met Ile Ser Gly Val Leu Tyr Ala Leu Leu Ala Gly Leu
1               5                   10                  15

Met Trp Gly Leu Ile Phe Val Gly Pro Leu Ile Val Pro Glu Tyr Pro
            20                  25                  30

Ala Met Leu Gln Ser Met Gly Arg Tyr Leu Ala Leu Gly Leu Ile Ala
        35                  40                  45

Leu Pro Ile Ala Trp Leu Gly Arg Val Arg Leu Arg Gln Leu Ala Arg
    50                  55                  60

Arg Asp Trp Leu Thr Ala Leu Met Leu Thr Met Met Gly Asn Leu Ile
65                  70                  75                  80

Tyr Tyr Phe Cys Leu Ala Ser Ala Ile Gln Arg Thr Gly Ala Pro Val
                85                  90                  95

Ser Thr Met Ile Ile Gly Thr Leu Pro Val Val Ile Pro Val Phe Ala
            100                 105                 110

Asn Leu Leu Tyr Ser Gln Arg Asp Gly Lys Leu Ala Trp Gly Lys Leu
        115                 120                 125

Ala Pro Ala Leu Ile Cys Ile Gly Ile Gly Leu Ala Cys Val Asn Ile
    130                 135                 140

Ala Glu Leu Asn His Gly Leu Pro Asp Phe Asp Trp Ala Arg Tyr Thr
145                 150                 155                 160

Ser Gly Ile Val Leu Ala Leu Val Ser Val Val Cys Trp Ala Trp Tyr
                165                 170                 175

Ala Leu Arg Asn Ala Arg Trp Leu Arg Glu Asn Pro Asp Lys His Pro
            180                 185                 190

Met Met Trp Ala Thr Ala Gln Ala Leu Val Thr Leu Pro Val Ser Leu
        195                 200                 205

Ile Gly Tyr Leu Val Ala Cys Tyr Trp Leu Asn Thr Gln Thr Pro Asp
    210                 215                 220

Phe Ser Leu Pro Phe Gly Pro Arg Pro Leu Val Phe Ile Ser Leu Met
225                 230                 235                 240

Val Ala Ile Ala Val Leu Cys Ser Trp Val Gly Ala Leu Cys Trp Asn
                245                 250                 255

Val Ala Ser Gln Leu Leu Pro Thr Val Ile Leu Gly Pro Leu Ile Val
            260                 265                 270

Phe Glu Thr Leu Ala Gly Leu Leu Tyr Thr Phe Leu Leu Arg Gln Gln
        275                 280                 285

Met Pro Pro Leu Met Thr Leu Ser Gly Ile Ala Leu Leu Val Ile Gly
    290                 295                 300

Val Val Ile Ala Val Arg Ala Lys Pro Glu Lys Pro Leu Thr Glu Ser
305                 310                 315                 320

Val Ser Glu Ser

<210>  3
<211>  966
<212>  DNA

<213>  Salmonella typhimurium


<220>
<221>  CDS
<222>  (1)..(966)
<223>  codierende Region von ytfF

<400>  3
atg att agc ggc gtg cta tac gcc ctg ctg gca ggg cta atg tgg gga        48
Met Ile Ser Gly Val Leu Tyr Ala Leu Leu Ala Gly Leu Met Trp Gly
1               5                   10                  15

ttg att ttc gtc ggc ccg tta atc gtg ccg gat tac ccg gcc ctc tta        96
Leu Ile Phe Val Gly Pro Leu Ile Val Pro Asp Tyr Pro Ala Leu Leu
            20                  25                  30

cag tca atg gga cgc tat ctg gcg ctg ggg ctg att gcc ctg ccg att       144
Gln Ser Met Gly Arg Tyr Leu Ala Leu Gly Leu Ile Ala Leu Pro Ile
        35                  40                  45

gcc tgg tta ggc cgc acg cgg tta cgc cag tta acg cgc aaa gac tgg       192
Ala Trp Leu Gly Arg Thr Arg Leu Arg Gln Leu Thr Arg Lys Asp Trp
    50                  55                  60

tcc acc gcc ctg tcg ctg acc ctg atg ggc aat gta atc tat tac gcc       240
Ser Thr Ala Leu Ser Leu Thr Leu Met Gly Asn Val Ile Tyr Tyr Ala
65                  70                  75                  80

tgc ctc gcc agc gcc att cag cga acc ggc gcg ccg gtg tcg acg atg       288
Cys Leu Ala Ser Ala Ile Gln Arg Thr Gly Ala Pro Val Ser Thr Met
                85                  90                  95

atc atc ggg aca ttg ccg gtc gtc att ccg ctt ttc gct aac gtt ctc       336
Ile Ile Gly Thr Leu Pro Val Val Ile Pro Leu Phe Ala Asn Val Leu
            100                 105                 110

tat agc caa cga gac ggc aga tta tcc tgg cgg cgt tta gcg ccc gcg       384
Tyr Ser Gln Arg Asp Gly Arg Leu Ser Trp Arg Arg Leu Ala Pro Ala
        115                 120                 125

ctg gcg ctt atc ggt ttt ggc ctg ctg tgc gtc aat att gct gaa ctt       432
Leu Ala Leu Ile Gly Phe Gly Leu Leu Cys Val Asn Ile Ala Glu Leu
    130                 135                 140

agc cag gga ttg tcc gat gtt agc ggc tgg cgc tat ggt tca ggc att       480
Ser Gln Gly Leu Ser Asp Val Ser Gly Trp Arg Tyr Gly Ser Gly Ile
145                 150                 155                 160

gtg ctg gcg ctg att tcc gta gcg tgc tgg gca tgg tat gcg ctg cgc       528
Val Leu Ala Leu Ile Ser Val Ala Cys Trp Ala Trp Tyr Ala Leu Arg
                165                 170                 175

aac gcc cgc tgg ctg cgg gaa aat ccc gac aaa cat ccc atg atg tgg       576
Asn Ala Arg Trp Leu Arg Glu Asn Pro Asp Lys His Pro Met Met Trp
            180                 185                 190

gca acg gcg cag gcg ctg gtt acg ctg ccg gtc tcg tta atc ggc tat       624
Ala Thr Ala Gln Ala Leu Val Thr Leu Pro Val Ser Leu Ile Gly Tyr
        195                 200                 205

gcg gcg gtc tgt ctg tgg ctg agt atc cag aaa ccg ctg ttt acc ctg       672
Ala Ala Val Cys Leu Trp Leu Ser Ile Gln Lys Pro Leu Phe Thr Leu
    210                 215                 220

ccc ttt ggc ccg cat ccc gcg ctg ttt atc ggt ctg atg atc gcc atc       720
Pro Phe Gly Pro His Pro Ala Leu Phe Ile Gly Leu Met Ile Ala Ile
225                 230                 235                 240

gcg gtg ctg tgt tcc tgg gtg ggc gcg ctg tgc tgg aat atc gcc agc       768
Ala Val Leu Cys Ser Trp Val Gly Ala Leu Cys Trp Asn Ile Ala Ser
                245                 250                 255

caa aaa ttg cca acc gtt att tta ggg ccg ctg att gtt ttt gag acg       816
Gln Lys Leu Pro Thr Val Ile Leu Gly Pro Leu Ile Val Phe Glu Thr
            260                 265                 270

ttg gcg gga ctg ctc tat acc ttc att ctg cgc cag gaa atc ccg cca       864
Leu Ala Gly Leu Leu Tyr Thr Phe Ile Leu Arg Gln Glu Ile Pro Pro
        275                 280                 285

ttc ctg acg tta agc ggc att gcg ctc ctg gtg gcg ggc gtg gtc att       912
Phe Leu Thr Leu Ser Gly Ile Ala Leu Leu Val Ala Gly Val Val Ile
    290                 295                 300

gcc gtg aga gcg aaa ccg gaa aaa gcg acc gtc gtt ccg gtc acg gaa       960
Ala Val Arg Ala Lys Pro Glu Lys Ala Thr Val Val Pro Val Thr Glu
305                 310                 315                 320

cgc taa                                                               966
Arg

<210>  4
<211>  321
<212>  PRT
<213>  Salmonella typhimurium

<400>  4

Met Ile Ser Gly Val Leu Tyr Ala Leu Leu Ala Gly Leu Met Trp Gly
1               5                   10                  15

Leu Ile Phe Val Gly Pro Leu Ile Val Pro Asp Tyr Pro Ala Leu Leu
            20                  25                  30

Gln Ser Met Gly Arg Tyr Leu Ala Leu Gly Leu Ile Ala Leu Pro Ile
        35                  40                  45

Ala Trp Leu Gly Arg Thr Arg Leu Arg Gln Leu Thr Arg Lys Asp Trp
    50                  55                  60

Ser Thr Ala Leu Ser Leu Thr Leu Met Gly Asn Val Ile Tyr Tyr Ala
65                  70                  75                  80

Cys Leu Ala Ser Ala Ile Gln Arg Thr Gly Ala Pro Val Ser Thr Met
                85                  90                  95

Ile Ile Gly Thr Leu Pro Val Val Ile Pro Leu Phe Ala Asn Val Leu
            100                 105                 110

Tyr Ser Gln Arg Asp Gly Arg Leu Ser Trp Arg Arg Leu Ala Pro Ala
        115                 120                 125

Leu Ala Leu Ile Gly Phe Gly Leu Leu Cys Val Asn Ile Ala Glu Leu
    130                 135                 140

Ser Gln Gly Leu Ser Asp Val Ser Gly Trp Arg Tyr Gly Ser Gly Ile
145                 150                 155                 160

Val Leu Ala Leu Ile Ser Val Ala Cys Trp Ala Trp Tyr Ala Leu Arg
                165                 170                 175

Asn Ala Arg Trp Leu Arg Glu Asn Pro Asp Lys His Pro Met Met Trp
            180                 185                 190

Ala Thr Ala Gln Ala Leu Val Thr Leu Pro Val Ser Leu Ile Gly Tyr
        195                 200                 205

Ala Ala Val Cys Leu Trp Leu Ser Ile Gln Lys Pro Leu Phe Thr Leu
    210                 215                 220

Pro Phe Gly Pro His Pro Ala Leu Phe Ile Gly Leu Met Ile Ala Ile
225                 230                 235                 240

Ala Val Leu Cys Ser Trp Val Gly Ala Leu Cys Trp Asn Ile Ala Ser
                245                 250                 255

Gln Lys Leu Pro Thr Val Ile Leu Gly Pro Leu Ile Val Phe Glu Thr
            260                 265                 270

Leu Ala Gly Leu Leu Tyr Thr Phe Ile Leu Arg Gln Glu Ile Pro Pro
        275                 280                 285

Phe Leu Thr Leu Ser Gly Ile Ala Leu Leu Val Ala Gly Val Val Ile
    290                 295                 300

Ala Val Arg Ala Lys Pro Glu Lys Ala Thr Val Val Pro Val Thr Glu
305                 310                 315                 320

Arg

<210>  5
<211>  34
<212>  DNA
<213>  Synthetische Sequenz

<220>
<221>  Primer
<222>  (1)..(34)
<223>  ytfF_No

<400>  5
cagtacgcgg ccgctcatgc agcatggtca ggta                                34

<210>  6
<211>  53
<212>  DNA
<213>  Synthetische Sequenz

<220>
<221>  Primer
<222>  (1)..(53)
<223>  ytfF_Ni

<400>  6
cacgcaataa ccttcacact ccaaatttat aacatcgcgc tgttagtgat tgg          53


<210>  7
<211>  53
<212>  DNA
<213>  Synthetische Sequenz


<220>
<221>  Primer
<222>  (1)..(53)
<223>  ytfF_Ci

<400>  7
gttataaatt tggagtgtga aggttattgc gtgctggcat ccattgatct gtc          53


<210>  8
<211>  33
<212>  DNA
<213>  Synthetische Sequenz


<220>
<221>  Primer
<222>  (1)..(33)
<223>  ytfF_Co

<400>  8
tagtcgcggc cgcagcactg gaacacggcg tat                                33


<210>  9
<211>  1139
<212>  DNA
<213>  Escherichia coli


<220>
<221>  gemischtes Merkmal
<222>  (1)..(1139)
<223>  mutagene DNA  delta-ytfF-ORF

<220>
<221>  gemischtes Merkmal
<222>  (524)..(526)
<223>  Startcodon des delta-ytfF-ORF

<220>
<221>  gemischtes Merkmal
<222>  (524)..(530)
<223>  5'-Region des delta-ytfF-ORF

<220>
<221>  gemischtes Merkmal
<222>  (531)..(563)
<223>  technische DNA

<220>

<221>  gemischtes Merkmal
<222>  (564)..(647)
<223>  3'-Region des delta-ytfF-ORF

<220>
<221>  gemischtes Merkmal
<222>  (645)..(647)
<223>  Stoppcodon des delta-ytfF-ORF

<400>  9
tagtcgcggc cgcagcactg gaacacggcg tatttatcgg tgcggcgggc gatggcaaaa      60

tcgcctcagc aacgcgggca gattatgcgg cagctgcggc acgcgtgatt agcgaagccg     120

gtcacgaagg caaggtttac gaactggcgg gcgatagtgc ctggacgttg acacagttag     180

cggcagagct gaccaaacag agcggcaaac aggttaccta tcaaaatctg agcgaagccg     240

atttcgccgc ggcactgaaa agcgtcggac tgcccgacgg actggcggat atgctggcgg     300

attctgacgt tggcgcatcg aaaggcggtc tgtttgatga cagcaaaaca cttagcaaat     360

tgattggcca cccaacgaca acgttagccg aaagcgtaag ccatcttttt aatgttaata     420

actagttaat taaagtggca tcctcccgca tcctctctga taatgacggg atgccgggag     480

caatcatgtc tgcttcctga actttcttct gacagatcaa tggatgccag cacgcaataa     540

ccttcacact ccaaatttat aacatcgcgc tgttagtgat tggcgtggtc atcgcggtca     600

gagcaaaacc agaaaaacct ttaactgaat ctgtctcaga aagttgacac gctggcagtg     660

agttaaataa gcctctgcta cgtaagggtt atagcttttg ccttaaagat gcatttaaaa     720

tacatcttat cttattaaga atgaggtatc agctatggct tatcgcgacc aacctttagg     780

tgaactggcg ctctctattc ctcgcgcttc agctctgttt cgtaaatatg atatggatta     840

ctgctgtggc ggtaagcaga cgctggcgcg cgcggcggca cgtaaagaac tggatgttga     900

ggtcattgaa gctgaactgg caaagctcgc tgaacaaccg attgagaaag actggcgtag     960

cgccccgctg gcagaaatca tcgaccatat catcgtgcgc taccacgatc gtcaccgcga    1020

gcaactgccg gagctgattc tgcaagcgac taaagtcgag cgcgttcacg ccgacaaacc    1080

gagcgtgcca aaagggctga caaaatacct gaccatgctg catgagcggc cgcgtactg     1139


<210>  10
<211>  2006
<212>  DNA
<213>  Escherichia coli


<220>
<221>  CDS
<222>  (594)..(1526)
<223>  thrB-Gen

<400>  10
tgcaggtgat gaattgatga agttctccgg cattctttct ggttcgcttt cttatatctt      60

cggcaagtta gacgaaggca tgagtttctc cgaggcgacc acgctggcgc gggaaatggg     120

ttataccgaa ccggacccgc gagatgatct ttctggtatg gatgtggcgc gtaaactatt     180

gattctcgct cgtgaaacgg gacgtgaact ggagctggcg gatattgaaa ttgaacctgt     240

gctgcccgca gagtttaacg ccgagggtga tgttgccgct tttatggcga atctgtcaca 300

actcgacgat ctctttgccg cgcgcgtggc gaaggcccgt gatgaaggaa aagttttgcg 360

ctatgttggc aatattgatg aagatggcgt ctgccgcgtg aagattgccg aagtggatgg 420

taatgatccg ctgttcaaag tgaaaaatgg cgaaaacgcc ctggccttct atagccacta 480

ttatcagccg ctgccgttgg tactgcgcgg atatggtgcg ggcaatgacg ttacagctgc 540

cggtgtcttt gctgatctgc tacgtaccct ctcatggaag ttaggagtct gac atg 596
Met
1

gtt aaa gtt tat gcc ccg gct tcc agt gcc aat atg agc gtc ggg ttt 644
Val Lys Val Tyr Ala Pro Ala Ser Ser Ala Asn Met Ser Val Gly Phe
            5                   10                  15

gat gtg ctc ggg gcg gcg gtg aca cct gtt gat ggt gca ttg ctc gga 692
Asp Val Leu Gly Ala Ala Val Thr Pro Val Asp Gly Ala Leu Leu Gly
        20                  25                  30

gat gta gtc acg gtt gag gcg gca gag aca ttc agt ctc aac aac ctc 740
Asp Val Val Thr Val Glu Ala Ala Glu Thr Phe Ser Leu Asn Asn Leu
    35                  40                  45

gga cgc ttt gcc gat aag ctg ccg tca gaa cca cgg gaa aat atc gtt 788
Gly Arg Phe Ala Asp Lys Leu Pro Ser Glu Pro Arg Glu Asn Ile Val
50                  55                  60                  65

tat cag tgc tgg gag cgt ttt tgc cag gaa ctg ggt aag caa att cca 836
Tyr Gln Cys Trp Glu Arg Phe Cys Gln Glu Leu Gly Lys Gln Ile Pro
                70                  75                  80

gtg gcg atg acc ctg gaa aag aat atg ccg atc ggt tcg ggc tta ggc 884
Val Ala Met Thr Leu Glu Lys Asn Met Pro Ile Gly Ser Gly Leu Gly
            85                  90                  95

tcc agt gcc tgt tcg gtg gtc gcg gcg ctg atg gcg atg aat gaa cac 932
Ser Ser Ala Cys Ser Val Val Ala Ala Leu Met Ala Met Asn Glu His
        100                 105                 110

tgc ggc aag ccg ctt aat gac act cgt ttg ctg gct ttg atg ggc gag 980
Cys Gly Lys Pro Leu Asn Asp Thr Arg Leu Leu Ala Leu Met Gly Glu
    115                 120                 125

ctg gaa ggc cgt atc tcc ggc agc att cat tac gac aac gtg gca ccg 1028
Leu Glu Gly Arg Ile Ser Gly Ser Ile His Tyr Asp Asn Val Ala Pro
130                 135                 140                 145

tgt ttt ctc ggt ggt atg cag ttg atg atc gaa gaa aac gac atc atc 1076
Cys Phe Leu Gly Gly Met Gln Leu Met Ile Glu Glu Asn Asp Ile Ile
                150                 155                 160

agc cag caa gtg cca ggg ttt gat gag tgg ctg tgg gtg ctg gcg tat 1124
Ser Gln Gln Val Pro Gly Phe Asp Glu Trp Leu Trp Val Leu Ala Tyr
            165                 170                 175

ccg ggg att aaa gtc tcg acg gca gaa gcc agg gct att tta ccg gcg 1172
Pro Gly Ile Lys Val Ser Thr Ala Glu Ala Arg Ala Ile Leu Pro Ala
        180                 185                 190

cag tat cgc cgc cag gat tgc att gcg cac ggg cga cat ctg gca ggc 1220
Gln Tyr Arg Arg Gln Asp Cys Ile Ala His Gly Arg His Leu Ala Gly
    195                 200                 205

ttc att cac gcc tgc tat tcc cgt cag cct gag ctt gcc gcg aag ctg 1268

Phe Ile His Ala Cys Tyr Ser Arg Gln Pro Glu Leu Ala Ala Lys Leu
210                 215                 220                 225

atg aaa gat gtt atc gct gaa ccc tac cgt gaa cgg tta ctg cca ggc      1316
Met Lys Asp Val Ile Ala Glu Pro Tyr Arg Glu Arg Leu Leu Pro Gly
                230                 235                 240

ttc cgg cag gcg cgg cag gcg gtc gcg gaa atc ggc gcg gta gcg agc      1364
Phe Arg Gln Ala Arg Gln Ala Val Ala Glu Ile Gly Ala Val Ala Ser
            245                 250                 255

ggt atc tcc ggc tcc ggc ccg acc ttg ttc gct ctg tgt gac aag ccg      1412
Gly Ile Ser Gly Ser Gly Pro Thr Leu Phe Ala Leu Cys Asp Lys Pro
        260                 265                 270

gaa acc gcc cag cgc gtt gcc gac tgg ttg ggt aag aac tac ctg caa      1460
Glu Thr Ala Gln Arg Val Ala Asp Trp Leu Gly Lys Asn Tyr Leu Gln
    275                 280                 285

aat cag gaa ggt ttt gtt cat att tgc cgg ctg gat acg gcg ggc gca      1508
Asn Gln Glu Gly Phe Val His Ile Cys Arg Leu Asp Thr Ala Gly Ala
290                 295                 300                 305

cga gta ctg gaa aac taa atgaaactct acaatctgaa agatcacaac             1556
Arg Val Leu Glu Asn
                310

gagcaggtca gctttgcgca agccgtaacc caggggttgg gcaaaaatca ggggctgttt   1616

tttccgcacg acctgccgga attcagcctg actgaaattg atgagatgct gaagctggat   1676

tttgtcaccc gcagtgcgaa gatcctctcg gcgtttattg gtgatgaaat cccacaggaa   1736

atcctggaag agcgcgtgcg cgcggcgttt gccttcccgg ctccggtcgc caatgttgaa   1796

agcgatgtcg gttgtctgga attgttccac gggccaacgc tggcatttaa agatttcggc   1856

ggtcgcttta tggcacaaat gctgacccat attgcgggtg ataagccagt gaccattctg   1916

accgcgacct ccggtgatac cggagcggca gtggctcatg ctttctacgg tttaccgaat   1976

gtgaaagtgg ttatcctcta tccacgaggc                                     2006

<210>  11
<211>  310
<212>  PRT
<213>  Escherichia coli

<400>  11

Met Val Lys Val Tyr Ala Pro Ala Ser Ser Ala Asn Met Ser Val Gly
1               5                   10                  15

Phe Asp Val Leu Gly Ala Ala Val Thr Pro Val Asp Gly Ala Leu Leu
            20                  25                  30

Gly Asp Val Val Thr Val Glu Ala Ala Glu Thr Phe Ser Leu Asn Asn
        35                  40                  45

Leu Gly Arg Phe Ala Asp Lys Leu Pro Ser Glu Pro Arg Glu Asn Ile
    50                  55                  60

Val Tyr Gln Cys Trp Glu Arg Phe Cys Gln Glu Leu Gly Lys Gln Ile

65                  70                  75                  80

Pro Val Ala Met Thr Leu Glu Lys Asn Met Pro Ile Gly Ser Gly Leu
                85                  90                  95

Gly Ser Ser Ala Cys Ser Val Val Ala Ala Leu Met Ala Met Asn Glu
            100                 105                 110

His Cys Gly Lys Pro Leu Asn Asp Thr Arg Leu Leu Ala Leu Met Gly
        115                 120                 125

Glu Leu Glu Gly Arg Ile Ser Gly Ser Ile His Tyr Asp Asn Val Ala
    130                 135                 140

Pro Cys Phe Leu Gly Gly Met Gln Leu Met Ile Glu Glu Asn Asp Ile
145                 150                 155                 160

Ile Ser Gln Gln Val Pro Gly Phe Asp Glu Trp Leu Trp Val Leu Ala
                165                 170                 175

Tyr Pro Gly Ile Lys Val Ser Thr Ala Glu Ala Arg Ala Ile Leu Pro
            180                 185                 190

Ala Gln Tyr Arg Arg Gln Asp Cys Ile Ala His Gly Arg His Leu Ala
        195                 200                 205

Gly Phe Ile His Ala Cys Tyr Ser Arg Gln Pro Glu Leu Ala Ala Lys
    210                 215                 220

Leu Met Lys Asp Val Ile Ala Glu Pro Tyr Arg Glu Arg Leu Leu Pro
225                 230                 235                 240

Gly Phe Arg Gln Ala Arg Gln Ala Val Ala Glu Ile Gly Ala Val Ala
                245                 250                 255

Ser Gly Ile Ser Gly Ser Gly Pro Thr Leu Phe Ala Leu Cys Asp Lys
            260                 265                 270

Pro Glu Thr Ala Gln Arg Val Ala Asp Trp Leu Gly Lys Asn Tyr Leu
        275                 280                 285

Gln Asn Gln Glu Gly Phe Val His Ile Cys Arg Leu Asp Thr Ala Gly
    290                 295                 300

Ala Arg Val Leu Glu Asn
305                 310

<210>  12
<211>  36
<212>  DNA
<213>  Escherichia coli

<220>
<221>  Primer
<222>  (1)..(36)
<223>  thrB_No

<400>  12
cagtacgcgg ccgctgcagg tgatgaattg atgaag                                36


<210>  13
<211>  55
<212>  DNA
<213>  Escherichia coli


<220>
<221>  Primer
<222>  (1)..(55)
<223>  thrB_Ni

<400>  13
cacgcaataa ccttcacact ccaaatttat aactaaccat gtcagactcc taact           55


<210>  14
<211>  53
<212>  DNA
<213>  Escherichia coli


<220>
<221>  Primer
<222>  (1)..(53)
<223>  thrB_Ci

<400>  14
gttataaatt tggagtgtga aggttattgc gtgcgacctt gttcgctctg tgt             53


<210>  15
<211>  35
<212>  DNA
<213>  Escherichia coli


<220>
<221>  Primer
<222>  (1)..(35)
<223>  thrB_Co

<400>  15
tagtcgcggc cgcgcctcgt ggatagagga taacc                                 35


<210>  16
<211>  183
<212>  DNA
<213>  Escherichia coli


<220>
<221>  gemischtes Merkmal
<222>  (1)..(183)
<223>  mutagene DNA  delta-thrB

<220>
<221>  gemischtes Merkmal

<222>  (1)..(3)
<223>  Startcodon des delta-thrB-Allels

<220>
<221>  gemischtes Merkmal
<222>  (1)..(11)
<223>  5'-Region des delta-thrB-Allels

<220>
<221>  gemischtes Merkmal
<222>  (12)..(29)
<223>  technische DNA

<220>
<221>  gemischtes Merkmal
<222>  (30)..(180)
<223>  3'-Region des delta-thrB-Allels

<220>
<221>  gemischtes Merkmal
<222>  (181)..(183)
<223>  Stoppcodon des delta-thrB-Allels

<400>  16
atggttagtt ataaatttgg agtgtgaagg ttattgcgtg cgaccttgtt cgctctgtgt     60

gacaagccgg aaaccgccca gcgcgttgcc gactggttgg gtaagaacta cctgcaaaat    120

caggaaggtt ttgttcatat ttgccggctg gatacggcgg gcgcacgagt actggaaaac    180

taa                                                                  183


<210>  17
<211>  20
<212>  DNA
<213>  Escherichia coli


<220>
<221>  Primer
<222>  (1)..(20)
<223>  thrA1

<400>  17
aaggtaacga ggtaacaacc                                                 20


<210>  18
<211>  20
<212>  DNA
<213>  Escherichia coli


<220>
<221>  Primer
<222>  (1)..(20)
<223>  thrA2

<400>  18
tcaaacccga cgctcatatt                                                 20